# EUROPEAN PATENT SPECIFICATION

(11) **EP 2 326 248 B1**
(45) Date of publication and mention of the grant of the patent: **08.11.2017**
(21) Application number: 09792256.1
(22) Date of filing: 04.09.2009
(51) Int. Cl.: A61B 6/00, A61B 6/10, A61B 6/02

(54) **INTEGRATED MULTI-MODE MAMMOGRAPHY/TOMOSYNTHESIS X-RAY SYSTEM**
INTEGRIERTES MULTIMODUS-MAMMOGRAPHIE/TOMOSYNTHESE-RÖNTGENSYSTEM
SYSTÈME DE RAYONS X POUR MAMMOGRAPHIE/TOMOSYNTHÈSE MULTI-MODE INTÉGRÉ

(30) Priority: 04.09.2008 US 94320 P
(43) Date of publication of application: 01.06.2011
(73) Proprietor: Hologic Inc., Marlborough, MA 01752 (US)
(72) Inventor: REN, Baorui, Andover, MA 01810 (US); SMITH, Andrew, Lexington, MA 02420 (US); JING, Zhenxue, Chadds Ford, PA 19317 (US); STEIN, Jay, Boston, MA 02116 (US)
(74) Representative: Hoyng Rokh Monegier LLP
(86) International application number: PCT/US2009/055981
(87) International publication number: WO 2010/028208

(56) References cited:
- WO-A2-2006/058160
- US-A1- 2008 112 534
- US-B2- 7 245 694

## Description

### Field of the invention

This patent specification pertains to x-ray mammography and, more specifically, to an integrated system for selectively carrying out x-ray mammography and/or tomosynthesis imaging and a method of using such a system.

### Background

X-ray mammography has long been a screening modality for breast cancer and other lesions, and also has been relied on for diagnostic and other purposes. For many years, the breast image was recorded on x-ray film but more recently digital x-ray image receptors have come into use, as in the Selenia™ mammography system available from Hologic Inc. of Bedford, MA and its division Lorad Corporation of Danbury, CT. For mammograms, a cone-shaped or pyramid-shaped x-ray beam passes through the compressed breast and forms a two-dimensional projection image. Any one of a number of orientations can be used, such as cranial-caudal (CC) or MLO (mediolateral-oblique) orientation. More recently, breast x-ray tomosynthesis has been proposed. The technology typically involves taking two-dimensional (2D) projection images of the immobilized breast at each of a number of angles of the x-ray beam relative to the breast and processing the resulting x-ray measurements to reconstruct images of breast slices that typically are in planes transverse to the x-ray beam axis, such as parallel to the image plane of a mammogram of the same breast. The range of angles is substantially less than in computerized tomography, i.e. substantially less than 180°, e.g. ±15°. Tomosynthesis technology is described in U.S. Patent Application Ser. No. 10/723,486 filed November 26, 2003; a prototype of a unit with at least some of the described features was shown at the 2003 Radiological Society of North America meeting in Chicago, Ill. Additional prototypes are in clinical testing in this country as of the filing of this patent specification. Other approaches to tomosynthesis also have been proposed: see, e.g., U.S. Patents Nos. 4,496,557, 5,051,904, 5,359,637, 6,289,235, and 6,647,092, published U.S. Patent Applications Nos. 2001/0038861, 2004/066882, 2004/0066884, and 2004/0066904, and Digital Clinical Reports, Tomosynthesis (GE Brochure 98-5493, 11/98). How to reconstruct tomosynthesis images is discussed in DG Grant, "Tomosynthesis: a three-dimensional imaging technique", IEEE Trans. Biomed. Engineering, Vol BME-19, #1, (January 1972), pp 20-28. See, also, U.S. Provisional Application Serial No. 60/628,516, filed November 15, 2004, and entitled "Matching geometry generation and display of mammograms and tomosynthesis images". Mammography systems can also be used in interventional procedures, such as biopsy, by adding a biopsy station (for example, the StereoLoc II™ Upright Stereotactic Breast Biopsy System, which is available from Hologic, Inc.).

In clinical use, it can be desirable for a number of reasons to assess both tomosynthesis images and conventional mammograms of the patient's breasts. For example, the decades of conventional mammograms have enabled medical professionals to develop valuable interpretation expertise. Mammograms may offer good visualization of microcalcifications, and can offer higher spatial resolution compared with tomosynthesis. Tomosynthesis images may have different desirable characteristics - e.g., they may offer better visualization of structures that can be obscured by overlying or underlying tissue in a conventional mammogram.

WO 2006/058160 A2 discloses a system for multi-mode breast x-ray imaging which comprises a compression arm assembly for compressing and immobilizing a breast for x-ray imaging, an x-ray tube assembly, and an x-ray image receptor is provided. The system is configured for a plurality of imaging protocols and modes.

US 2008/0112534 A1 discloses a mammographic imaging system that is optimized for use with a single fixed size flat panel digital image receptor. It accommodates compression devices (paddles) of varying sizes, and positions them properly in the field of view of the image receptor. When a compression paddle with size smaller than the field of view of the image receptor is used, the compression paddle can be shifted laterally in the direction parallel to the chest wall, so as to facilitate different views of different size breasts, and permit the image receptor to image as much of the desired tissue as possible. An automatic x-ray collimator restricts the x-ray illumination of the breast in accordance with compression paddle size and location in the field of view. An anti-scatter grid, mounted inside the image receptor enclosure, just below the top cover of the enclosure, can be retracted out of the field of view of the image receptor for use in magnification imaging.

US 7,245,694 B2 discloses a breast X-ray system according to the preamble of independent claim 1. The detector of the system disclosed in this document is adapted to relate the count of photons to a desired x-ray dose, but this count is not used for exposure control. While the existing and proposed systems for x-ray mammography and tomosynthesis offer many advantages, it is believed that a need still exists for further improvements to make mammography/tomosynthesis more useful, and that it is particularly desirable to make it possible to use the same system in different modes of operation and thereby reduce acquisition and operating costs and provide greater clinical value and convenience.

### Summary

The invention is defined in claim 1.

This patent specification describes examples of systems and methods for multi-mode breast x-ray imaging. A single system carries out breast imaging in modes that include standard mammography, diagnostic mammography, dynamic imaging such as with a contrast agent and at different x-ray energies, tomosynthesis imaging, combined standard and tomosynthesis imaging during a single breast compression, needle localization, and stereotactic imaging with a biopsy station mounted to the system.

According to an exemplary embodiment, a system for multi-mode breast x-ray imaging is provided which supports imaging using at least two imaging modes. At least one imaging mode differs from at least one other imaging mode by at least one imaging procedure selected from a group including, but not limited to, receptor motion, anti-scatter grid use, exposure control and patient shielding. Breast imaging using each of the different modes may occur during a single compression and image scan of the breast. The system supports a combination imaging mode wherein images are captured using at least two imaging modes during a single image scan and wherein each of the at least two imaging modes uses at least one different imaging procedure. For dual mode capture using a single scan, such an arrangement facilitates fast capture of a plurality of images of different types without decompression of a patient's breast. As a result, the quantity and quality of information available for screening and diagnosis is substantially increased without concomitant increase in examination time, or patient discomfort. For dual mode capture using different image scans, such an arrangement allows different imaging protocols to be implemented using a single imaging system. As a result, the quantity and quality of information available for screening and diagnosis is substantially increased without concomitant increase in examination equipment cost.

In an example of a system using the teachings of this patent specification, a compression arm assembly for compressing and immobilizing the breast for x-ray imaging, an x-ray tube assembly, and an x-ray image receptor can be angled differently relative to each other for different imaging protocols and modes. For example, in first mode such as a mammography mode the receptor may be positioned generally normal to the plane of the x-ray tube assembly. In second mode, such as a tomosynthesis mode, as the x-ray tube assembly rotates through a first angular range the receptor rocks through a second angular range that is less than the first angular range such that the relative position of the x-ray tube assembly and x-ray receptor is offset from normal. In a preferred embodiment, the x-ray tube assembly and the x-ray receptor rotate and rock with different angular displacements. As described above, the system supports a combination imaging mode wherein a plurality of images are captured during either during a single scan of the x-ray tube assembly or during multiple, temporally spaced scans, using at least two different imaging modes (where 'temporally spaced' may mean a different scan during the same compression of the breast or a different scan using a later compression of the same breast). In such an arrangement, the receptor moves into a plurality of positions relative to the x-ray assembly, at least one different position for each of the modes of imaging that are performed during the single scan.

A patient shield can be removably mounted to the compression arm assembly to provide a mechanical interlock against patient contact with the rotating x-ray tube assembly. In one embodiment, the patient shield may move to different positions for at least two different imaging modes of the multi-mode system. In an alternate embodiment, the patient shield may be removed and/or replaced for each of the different imaging modes of the multi-mode system.

A removable anti-scatter grid can be used that can cover the imaging area of the x-ray receptor in some modes but be removed for other modes. In a preferred embodiment, the anti-scatter grid is positioned parallel to and above the receptor in a first mode, and retracted in a second mode. However, as will be described in more detail below, other methods in addition to retraction may be used to remove the anti-scatter grid and the present invention is not limited to any particular method of grid removal.

According to another aspect of the invention, Automatic Exposure Controls (AECs) are varied in accordance with the breast density.

The exemplary system further includes a breast compression paddle that is laterally movable, under manual control or when motorized and operating under software control. The compression paddle can shift automatically depending on the view to be acquired. For example, the paddle can be centered on the x-ray receptor for a CC view, shifted to one lateral side of the receptor for an MLO view of one breast and to the other lateral side of the receptor for an MLO view of the other breast. The paddle can be automatically recognized by the system when mounted so that the shifts can be adjusted to the type of paddle.

The compression paddle can be easily removable from a support that has a mechanism for laterally moving the paddle and for allowing the paddle to tilt for better conformance with the breast for selected image modes but locking the paddle against tilt for other modes. With the movement mechanism in the support and not integral with the paddle, the paddle can be simple and inexpensive, and easy to mount to and remove from the support. A number of relatively inexpensive paddles of different sizes and shapes can be provided and conveniently interchanged to suit different procedures and patients.

### Brief description of the drawing

Fig. 1 is a perspective view of a gantry and an acquisition workstation in accordance with an example of the disclosed system.
Fig. 2 is an enlarged view of a portion of the system of Fig. 1, with a tube arm assembly in a rotated position.
Fig. 3 is a front elevation of the apparatus of Fig. 2.
Fig. 4 is a side view of a gantry with a biopsy station and a spacer, with schematic illustration of other mechanisms.
Fig. 5 is an enlarged view of a portion of Fig. 1.
Fig. 6 is a block diagram of the disclosed system when connected to other systems.
Fig. 7 is a flow chart illustrating a general work flow for the disclosed system.
Fig. 8 is a flow chart illustrating one of several examples of work flow for a standard mammography mode.

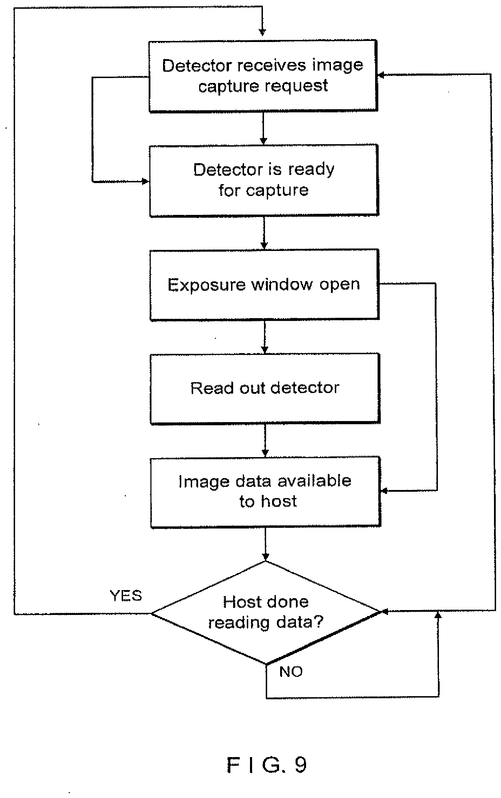

Fig 9 is a flow chart illustrating one of several examples of work flow for an image detector subsystem in the standard mammography mode.
Fig. 10 is a perspective view of the structure of Fig. 4.
Fig. 11 is similar to Fig. 2 but shows a tube arm assembly angled differently.
Fig. 12 is a front elevation of the structure of Fig. 11.
Fig. 13 is a flow chart illustrating one of several examples of work flow for a tomosynthesis mode.
Fig. 14 is a flow chart illustrating one of several examples of work flow for an image detector subsystem in the tomosynthesis mode.
Fig. 15 is a flow chart illustrating one of several examples of work flow for a combination mode.
Fig. 16 is a flow chart illustrating one of several examples of work flow for an image detector subsystem in the combination mode.
Fig. 17 is an enlarged side view of a structure for removably mounting a breast compression paddle.

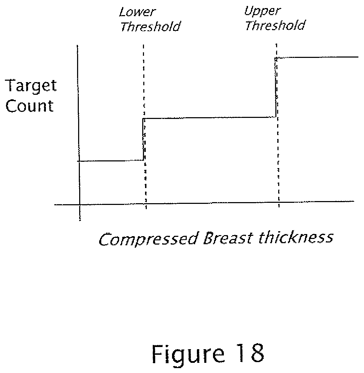

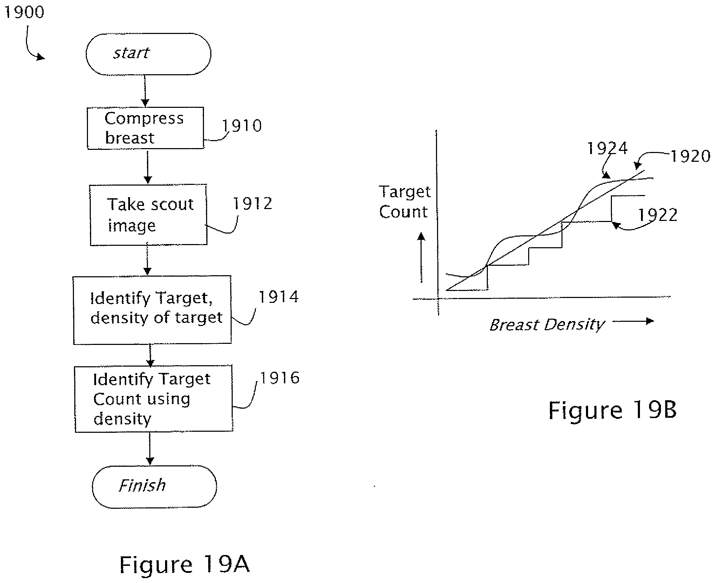

Figures 18,19A,19B illustrate Automatic Exposure Control tables.

### Detailed description

In describing examples and preferred embodiments illustrated in the drawings, specific terminology is employed for the sake of clarity. However, the disclosure of this patent specification is not intended to be limited to the specific terminology so selected and it is to be understood that each specific element includes all technical equivalents that operate in a similar manner.

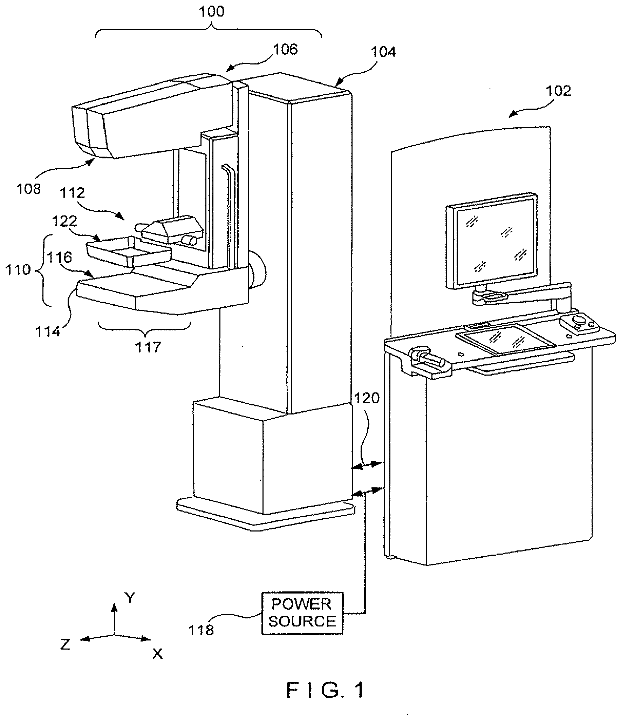

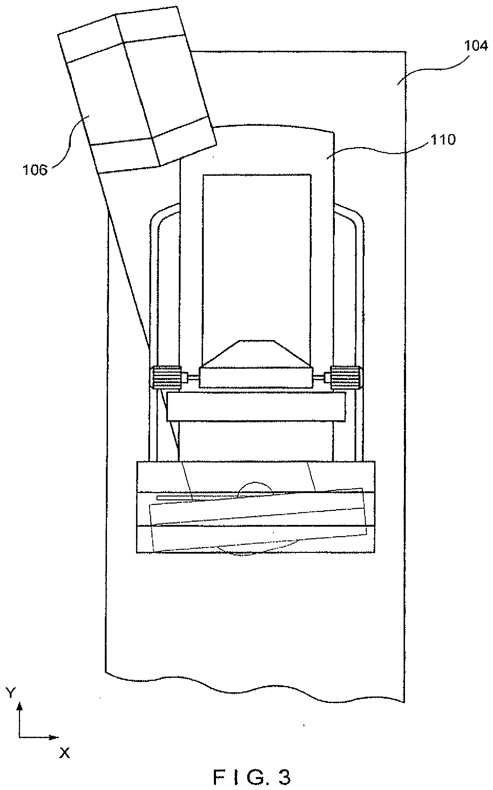

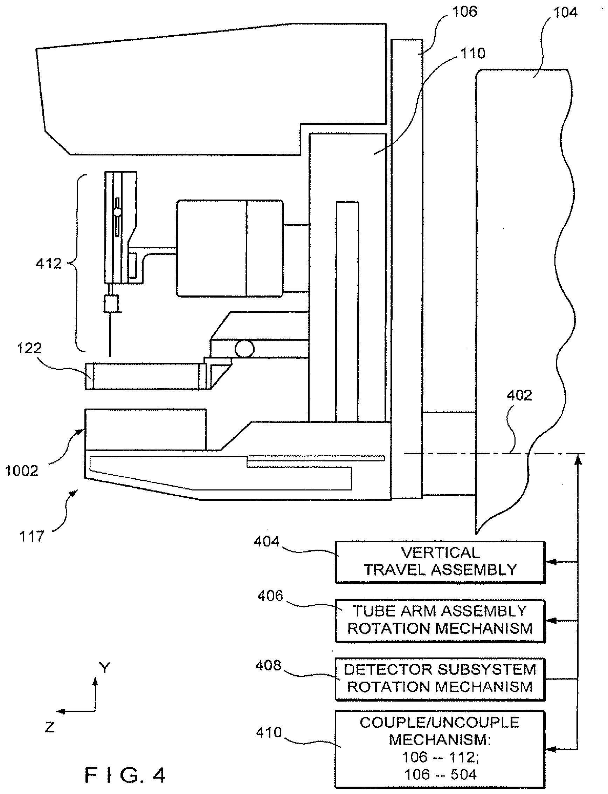

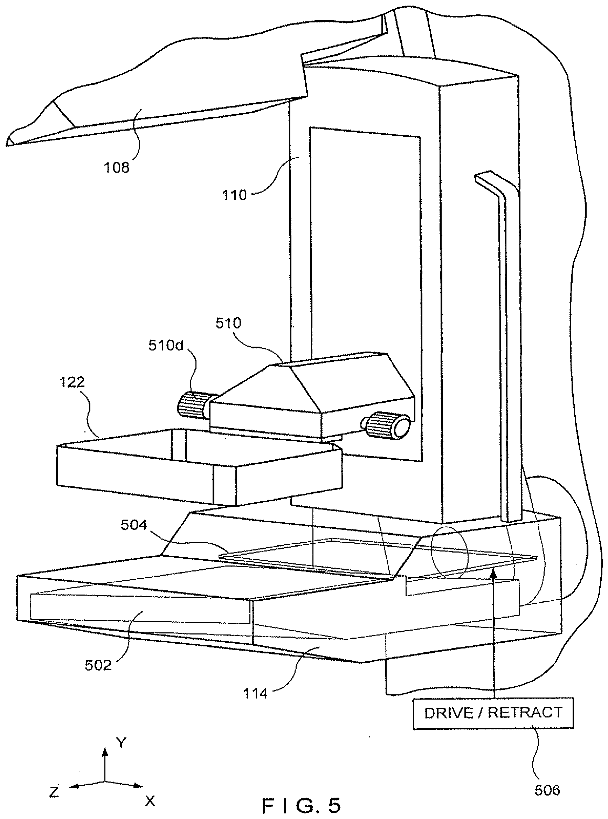

Figs. 1-6 illustrate a non-limiting example of a multi-mode mammography/ tomosynthesis system comprising a gantry 100 and a data acquisition work-station 102. Gantry 100 includes a housing 104 supporting a tube arm assembly 106 rotatably mounted thereon to pivot about a horizontal axis 402 (Fig. 4) and carrying an x-ray tube assembly 108. X-ray tube assembly 108 includes (1) an x-ray tube generating x-ray energy in a selected range, such as 20-50 kV, at mAs such as in the range 3-400 mAs, with focal spots such as a nominal size 0.3 mm large spot and nominal size 0.1 mm small spot (2) supports for multiple filters such as molybdenum, rhodium, aluminum, copper, and tin filters, and (3) an adjustable collimation assembly selectively collimating the x-ray beam from the focal spot in a range such as from 7x8 cm to 24x29 when measured at the image plane of an x-ray image receptor included in the system, at a maximum source-image distance such as 75 cm. Also mounted on housing 104, for rotation about the same axis 402, is a compression arm assembly 110 that comprises a compression plate 122 and a receptor housing 114 having an upper surface 116 serving as a breast plate and enclosing a detector subsystem system 117 comprising a flat panel x-ray receptor 502 (Fig. 5), a retractable anti-scatter grid 504 and a mechanism 506 for driving and retracting anti-scatter grid 504. Housing 104 also encloses the following components schematically illustrated in Fig. 4: a vertical travel assembly 404 for moving tube arm assembly 106 and compression arm assembly 110 up and down to accommodate a particular patient or imaging position, a tube arm assembly rotation mechanism 406 to rotate tube arm assembly 106 about axis 402 for different imaging positions, a detector subsystem rotation mechanism 408 for rotating components of detector subsystem 117 (such as x-ray receptor 502) about axis 402 to accommodate different operations modes, and couple/uncouple mechanism 410 to selectively couple or uncouple tube arm assembly 106 and compression arm assembly 110 to and from each other, and tube arm assembly 106 and detector subsystem 117 to and from each other. Housing 104 also encloses suitable motors and electrical and mechanical components and connections to implement the functions discussed here.

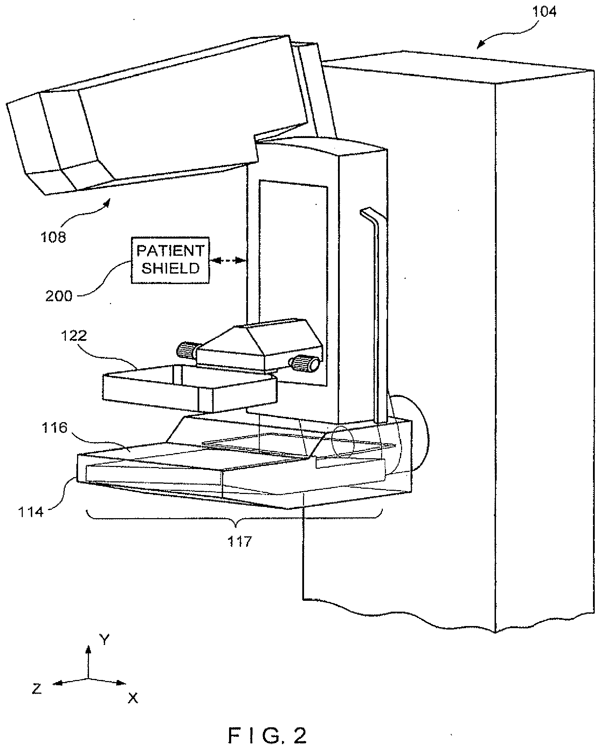

A patient shield 200, schematically illustrated in Fig. 2, can be secured to compression arm assembly 110 to provide a mechanical interlock against patient contact with the rotating x-ray tube arm assembly 106 and reduce patient interference with the x-ray image capture. One exemplary patient shield is a shifting patient shield that is positioned generally parallel to but outside the path of x-ray beams from source 108. The shifting face shield is supported by an extension arm that extends laterally from the compression arm assembly and permits movement of the face shield along a z axis. In various embodiments the patient shield may be removably secured either to the extension arm, compression arm assembly or the x-ray source assembly 108 to facilitate removal and/or replacement of shields for different imaging modes. In various embodiments, the face shield may be of fixed size, or may be adjustably sized. An example of an adjustable face shield is disclosed in U.S. 7,315,607, where the face shield is positioned to slide vertically along a y-axis, as indicated by arrow 201, up into the x-ray assembly. The vertical movement removes the face shield to allow for patient positioning as well as allows the shield to be selectively positioned to provide shields of different sizes for different imaging modes. Alternate embodiments are also envisioned wherein the face shield is programmed to shift laterally along an x-axis in response to a selection of a particular imaging mode. Such an embodiment is described in U.S. patent 7,245,694 issued July 17, 2007 to Hologic Inc.. In summary, it is anticipated that different shields or shield arrangements may be used in a system of the present invention which supports a combination imaging mode, wherein the shield is removable or moves, either automatically or manually, between at least two positions for at least two different imaging modes.

Work-station 102 comprises components similar to those in the Selenia™ mammography system, including a display screen (typically a flat panel display that may include touch-screen functionality), user interface devices such as a keyboard, possibly a touch-screen, and a mouse or trackball, and various switches and indicator lights and/or displays. Work-station 102 also includes computer facilities similar to those of the Selenia™ system (but adapted through hardware, firmware and software differences) for controlling gantry 100 and for processing, storing and displaying data received from gantry 100. A power generation facility for x-ray tube assembly 108 may be included in housing 104 or in work-station 102. A power source 118 powers work-station 102. Gantry 100 and work-station 102 exchange data and controls over a schematically illustrated connection 120.

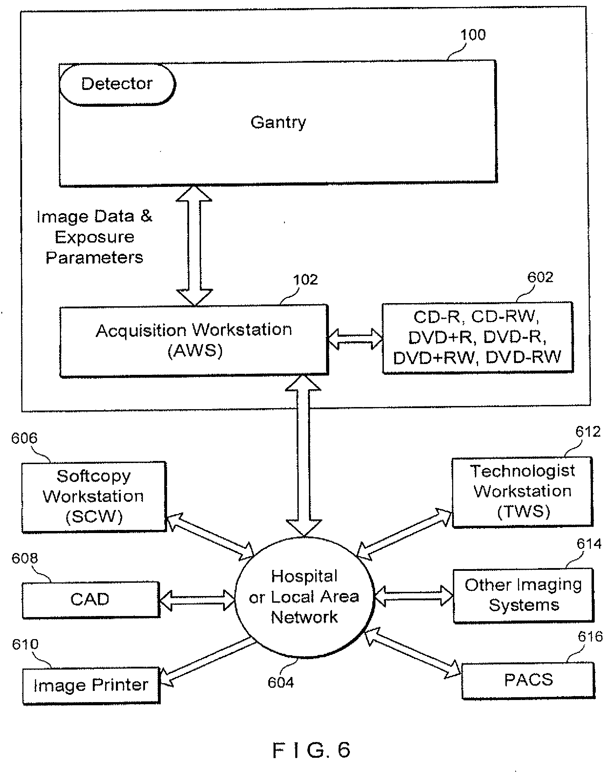

As illustrated in Fig. 6, additional storage facilities 602 can be connected to work-station 102, such as one or more optical disc drives for storing information such as images and/or for providing information to work-station 102 such as previously obtained images and software, or a local printer (not shown). In addition, the disclosed system can be connected to a hospital or local area or other network 604, and through the network to other systems such as a soft copy workstation 606, a CAD (Computer Aided Detection) station 608 for computer- processing mammography and/or tomosynthesis images to identify likely abnormalities, an image printer 610 for printing images, a technologist workstation 612, other imaging systems 614 such as other mammography systems or systems for other modalities for exchange of images and/or other information, and to a PACS (Picture Archiving) systems 616 for archiving images and other information and/or retrieving images and other information.

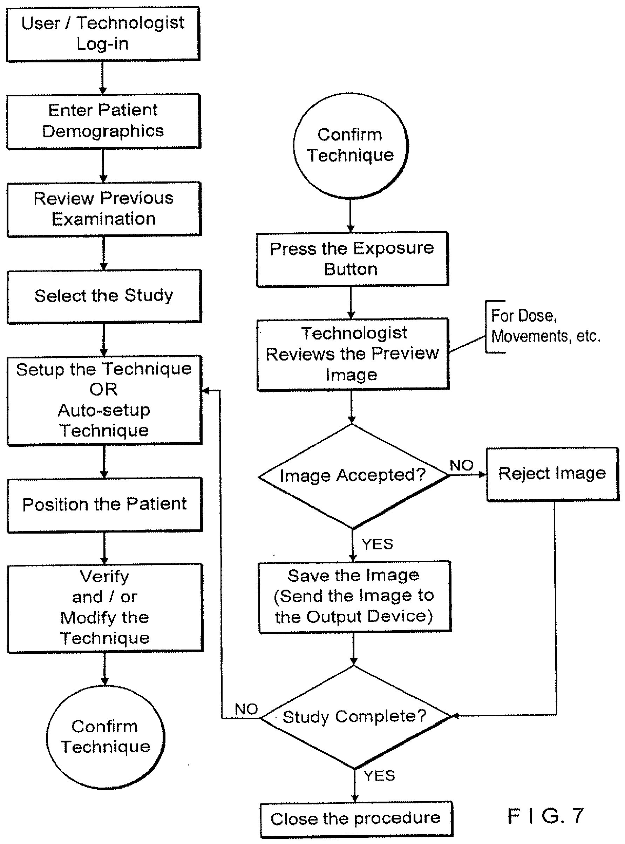

The illustrated system has several modes of operation. An example of typical workflow generally applicable for each mode is illustrated in Fig. 7, and several examples of operational modes are discussed below. Of course, this is only one example and workflow steps may be arranged differently. In all modes, the operator can perform x-ray exposure using manual setting of technic factors such as mA and mSec, or can use an Automatic Exposure Control (AEC) method as known in the art to set the exposure time, kV and filter modes for an image, for example by using a short, low-x-ray dose pre-exposure. Work-station 102 is set up to record the exposure technic information and associate it with the breast image for later review.

During an exemplary Automatic Exposure Control (AEC) method, a short (e.g., ∼5 msec) an x-ray is taken (either at a low dose, or full dose) and the image receptor's image is read by a computer process. This initial x-ray is often referred to as a 'scout' image. The computer process uses information from the scout image to identify the breast's radio-density and to calculate the correct final x-ray tube exposure voltage kVp, current mAs, and exposure time for delivering a desired x-ray dose be obtained prior to performing mammography or tomosynthesis.

In an alternate embodiment described in U.S. Patent 7,245,694, in a combination mammography/tomosynthesis system when a tomosynthesis scan follows a mammogram, the mammogram exposure data may be used to estimate tomosynthesis exposure techniques. In still another alternate embodiment, a first tomosynthesis image may be used to as the 'scout' image to estimate the appropriate exposure factors for the remaining images in the tomosynthesis sequence.

Lookup tables have historically been used to identify appropriate kVp and mAs for desired exposures using scout or mammogram images. Look-up tables are reliable in 2D mammography systems which position the x-ray source normal to the detector and utilize anti-scatter grids to minimize radiographic scatter. In such systems the detector count rate may be fixed, with exposure dosing being controlled by varying the kVp and mAs. However tomosynthesis systems which do not maintain a constant perpendicular relationship between the receptor and the x-ray source are not able to use an anti-scatter grid. The present invention recognizes that such systems may benefit from improved exposure control techniques having the ability to vary the target detector count to compensate for radiographic scatter, where the detector count relates a count of photons to a desired x-ray dose. The detector count may be varied in accordance with a thickness of the imaged breast. Thus thicker breasts, which experience a higher radiographic scatter, have a higher detector target count. Varying the detector count in accordance with breast thickness ensures that image quality of the breasts is maintained despite increased scatter.Although the table shows particular multipliers applied to the target counts for different breast thicknesses, it should be appreciated that the invention is not limited to any particular multipliers. Thus, when a multi-mode imaging system is used in combination mode, several different AEC techniques and/or parameters may be used during a single sweep of the x-ray tube assembly arm (or between different sweeps of the x-ray tube assembly).

In addition, although the table illustrates that a common kV, mA and count is provided for a given thickness, it is also envisioned that the AEC values may be customized for different imaging positions along the tomosynthesis path to accommodate for different types of radiographic scatter resulting from different x-ray source/receptor angular relationships, whether the imaging positions be associated with different imaging modes (such as mammography and tomosynthesis) or within the same imaging mode (i.e., different tomosynthesis imaging angles).

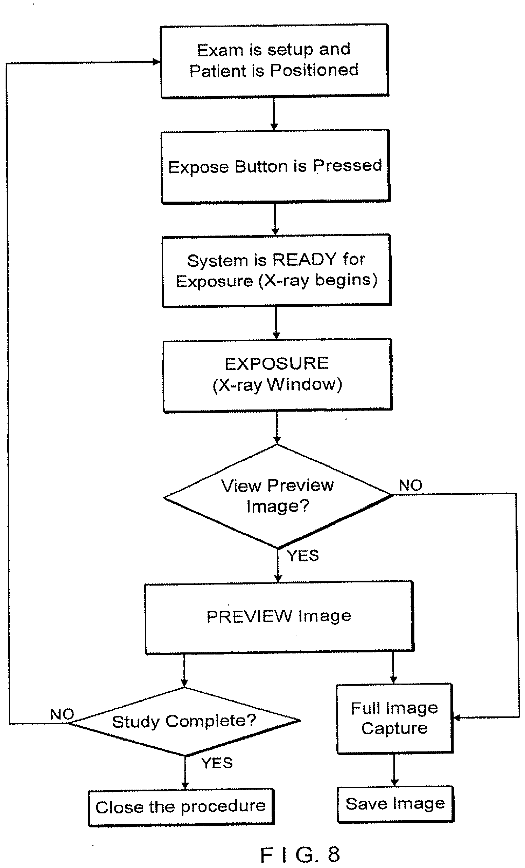

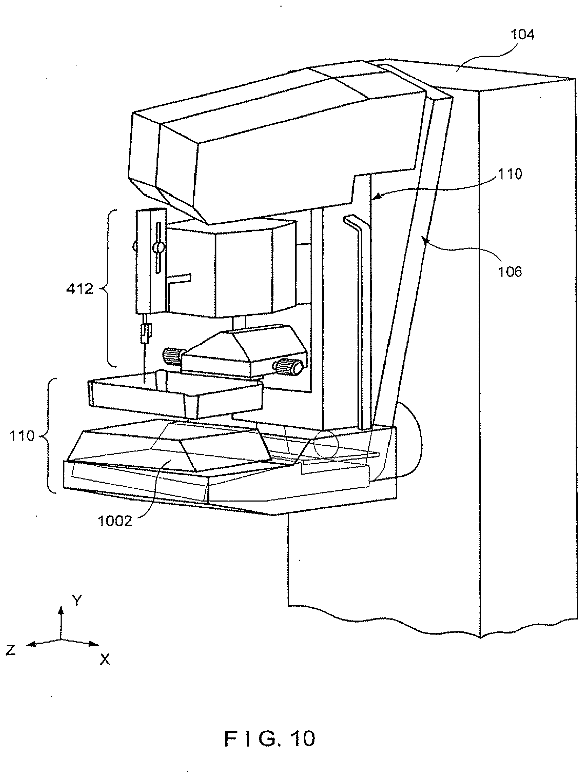

In standard mammography mode, typically used for screening mammography, tube arm assembly 106 and compression arm assembly 110 are coupled and locked together by 410 in a relative position such as seen in Fig. 1, such that an x-ray beam from x-ray tube assembly 108 illuminates x-ray receptor 502 when the patient's breast is compressed by compression device 112. In this mode, the system operates in a manner similar to said Selenia™ system to take a mammogram. Vertical travel assembly 404 and tube arm rotation mechanism 406 can make vertical adjustments to accommodate a patient, and can rotate tube arm assembly 106 and compression arm assembly 110 together as a unit about axis 402 for different image orientations such as for CC and for MLO images. For example, tube arm assembly 106 and compression arm assembly 110 can rotate between (-195°) and (+150°) about axis 402. As in the Selenia™ system, compression device 112 includes a compression paddle 122 that can move laterally, in a direction along the chest wall of a patient, to adjust for different imaging orientations. However, as described further below, the mechanism for supporting and moving compression paddle 122 is different. Typically, anti-scatter grid 504 is over x-ray receptor 502 in the standard mammography mode to reduce the effect of x-ray scatter. Fig. 8 illustrates a typical workflow for an exposure in standard mammography mode, and Fig. 10 illustrates an example of the operation of detector subsystem 117 in standard mammography. Of course, these are only examples; other workflow steps or orders of steps can be used instead.

In a diagnostic mode, the patient's breast can be spaced from upper surface 116, for example by an x-ray translucent spacer gantry 1002 (Fig. 10), with the system otherwise similar to Fig. 1, for a magnification of up to 1.8, for example. In this mode, as in standard mammography, tube arm assembly 106 and compression arm assembly 110 are locked to each other and can move up or down and rotate about axis 402 for different image orientation. A different spacer 1002 can be used for a different degree of magnification. Also, differently shaped or dimensioned compression paddles 122 can be used for different breast compression effects. The x-ray tube in x-ray tube assembly 108 can be set to a smaller focal spot size to improve a diagnostic image. In this mode, anti-scatter grid 504 typically is retracted or otherwise removed when magnification is used such that grid 504 is completely out of the image. The user can elect not to use a spacer 1002 in diagnostic imaging, in which case anti-scatter grid 504 can be used over the entire image.

In a dynamic imaging mode, a number of breast images are taken while the patient's breast remains compressed. In one technique, an agent such as iodine is injected into the patient and after a suitable waiting time such as about one minute for a maximum uptake, two images breast are taken in rapid succession, for example one at an x-ray energy just above the K-edge of iodine and one at an energy just below the K-edge. Alternatively, a succession of breast images can be taken at a single x-ray energy band or bands just above and below the K-edge, or at another x-ray energy range, to track the uptake of agent over time. Another technique adds taking a baseline breast image before or soon after injecting the agent and using it together with later breast images to generate subtraction images that provide better visualization of anatomy that may be of interest. Still another dynamic imaging mode technique comprises injecting a contrast agent and taking a succession of images over a period such as 5-7 minutes, for example one image every minute, and processing the image data to generate for each pixel, or at least for each pixel of interest, a histogram of the change in the pixel value, to thereby use the manner in which pixel values change to differential abnormal tissue. For this mode, work-station 102 can store preset data that commands gantry 100 and work-station 102 to take a desired sequence of images for the dynamic mode technique selected by the operator, such that the command data sets the appropriate parameters such as x-ray energy, dose, timing of images, etc. Alternatively, such processing to assess changes in pixel values can be done for a region of interest rather than over individual pixels, to produce information such as a measure of changes in the average pixel values in the region of interest.

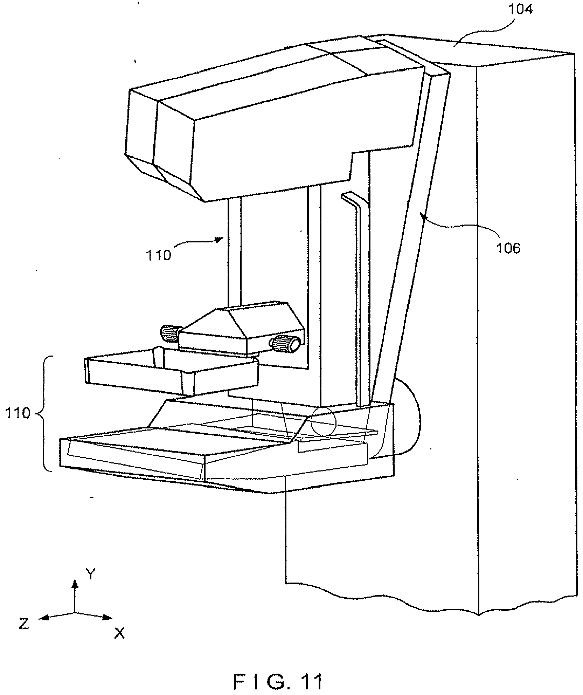

In tomosynthesis mode, tube arm assembly 106 and compression arm assembly 110 are decoupled by unit 410 such that compression arm assembly 110 stays in one position, compressing the patient's breast, while tube arm assembly 106 rotates about axis 402, for example between the position illustrated in Fig. 2 to that illustrated in Fig. 11, or ±15° relative to compression arm assembly 110. In one embodiment, during tomosynthesis mode the x-ray tube assembly rotates in an arc shaped path within a plane although this is not a requirement of the invention. Other tomosynthesis embodiments wherein the x-ray tube moves in a non-arc shaped path, for example through movement of the source outside of the plane of the x-ray assembly, or alternatively movement of the x-ray source vertically within the plane, are also envisioned.

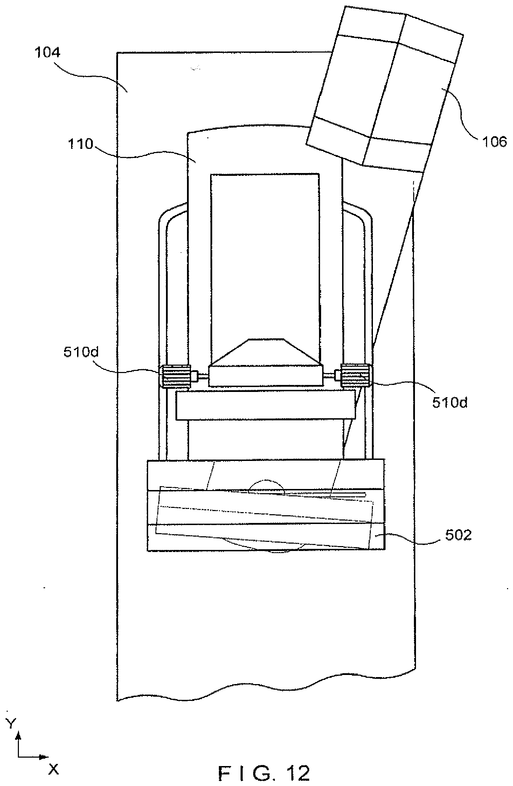

Tomosynthesis can be carried out for different image orientations, so that compression arm assembly 110 can be rotated about axis 402 (alone or together with assembly 106) for a desired image orientation and locked in place, and then tube arm assembly 106 can be rotated relative to that position of compression arm assembly 110 for tomosynthesis imaging over ±15° or some other desired angular range. In one example, 11 images are taken during an angular sweep of tube arm assembly 106, one every approximately 3°. However, a different number of images can be taken, for example up to 21 during a single sweep. For tomosynthesis images, the x-ray tube in x-ray tube assembly 108 continuously rotates and the x-ray tube is pulsed for each image, for example, for x-ray energy pulses each lasting approximately 100 mSec, although pulses of different duration can be selected. Alternatively, the rotational motion can stop for taking each image, or continuous motion without pulsing can be used (and the timing of data measurements relied to define pixel values). As seen in Figs. 2, 3, 5, 11 and 12, in this mode mechanism 506 fully retracts anti-scatter grid 504 away from x-ray receptor 502 so grid 504 is out of the image. Alternate methods of removing the anti-scatter grid from the image, such as ejecting the grid out of a side of the receptor housing 114 or otherwise accessing and removing the grid are also contemplated by this invention.

Also as seen in these Figs., while the breast remains immobilized in compression arm assembly 110 during the angular sweep of tube arm assembly 106, in one embodiment the x-ray receptor 502 rocks within receptor housing 114. In this rocking motion, controlled by unit 408 (Fig. 4), a line normal to the image face of x-ray receptor 502 may keep pointing to the focal spot of the x-ray tube in x-ray tube assembly 108. Alternatively, the rotation of tube arm assembly 106 and rocking of x-ray receptor 502 can be through different angles; for example, tube arm assembly 106 can rotate through 15° while x-ray receptor 502 rocks through 5°, i.e. the rocking angle can be an amount one-third that of assembly 108. In general, the receptor 502 rocks in concert with, but with a smaller angular displacement, from the x-ray tube assembly. Although a one-third relationship has been described, the present invention is not limited to any particular fractional angular relationship between the assembly and the receptor, and the present invention envisions systems wherein the detector is stationary during imaging.

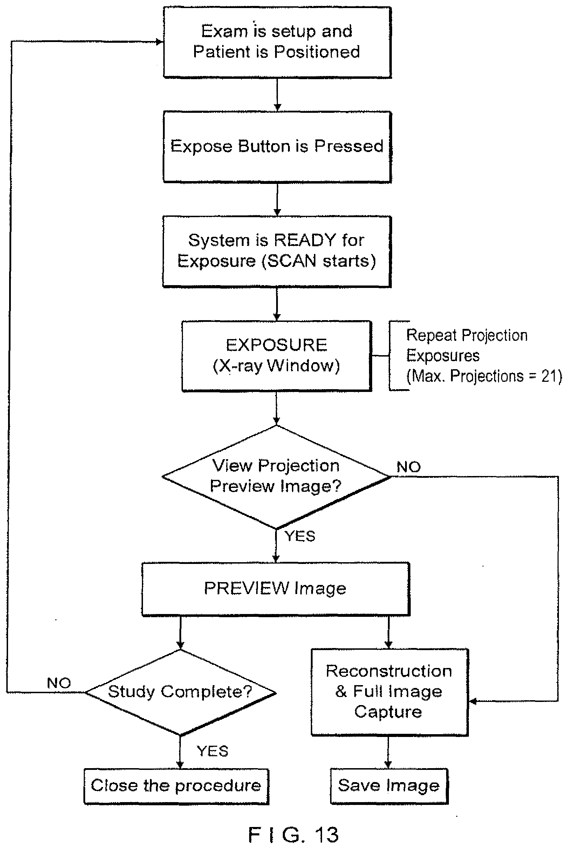

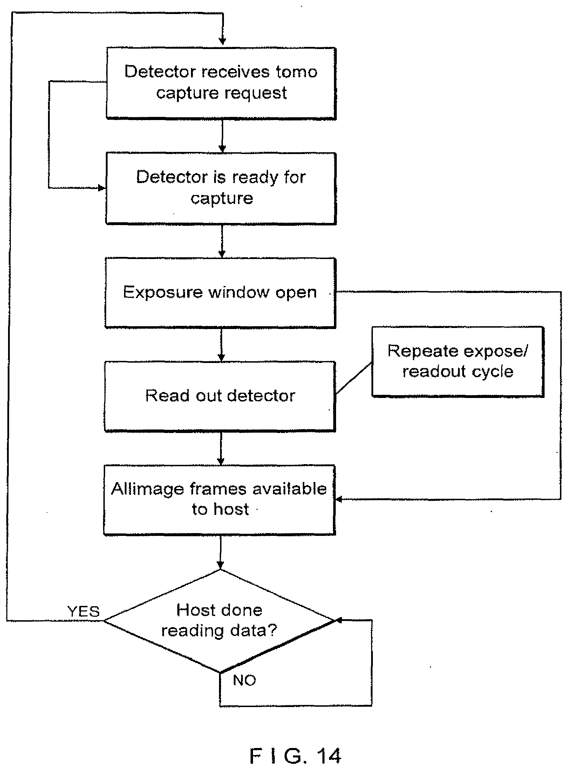

Synchronous rotation of tube arm assembly 106 and rocking of x-ray receptor 502 can be achieved by controlling separate motors for each or, alternatively, through using a motor to drive tube arm assembly 106 and a mechanical coupling between the rotation of tube arm assembly 106 and rocking of x-ray receptor 502. Image data can be obtained and processed into tomosynthesis images for display and/or storage as described for example in co-pending patent application Ser. No. 10/723,486 or in U.S Provisional Application No. 60/628,516, filed November 15, 2004. Fig. 13 illustrates a typical workflow for tomosynthesis mode operation, and Fig. 14 illustrates an example of the operation of detector subsystem 117 in that mode. Again, these are only examples, and other steps or orders of steps can be used instead.

It should be noted that although in preferred embodiments the receptor rocks such that it is positioned offset from normal to the x-ray source, this is not a requirement of the invention. Alternate embodiments where the receptor rotates along the same angular displacement and synchronous to the x-ray source are also contemplated. In addition, embodiments where the x-ray receptor moves linearly or laterally in a plane, rather than rocking, are within the scope of the present invention. In addition, in embodiments wherein the x-ray source assumes a non-arc shaped path, systems wherein the receptor rotates or tilts in a manner related to the x-ray source movement are contemplated by the present invention. Of course other embodiments wherein the detector remains stationary are also contemplated by this invention.

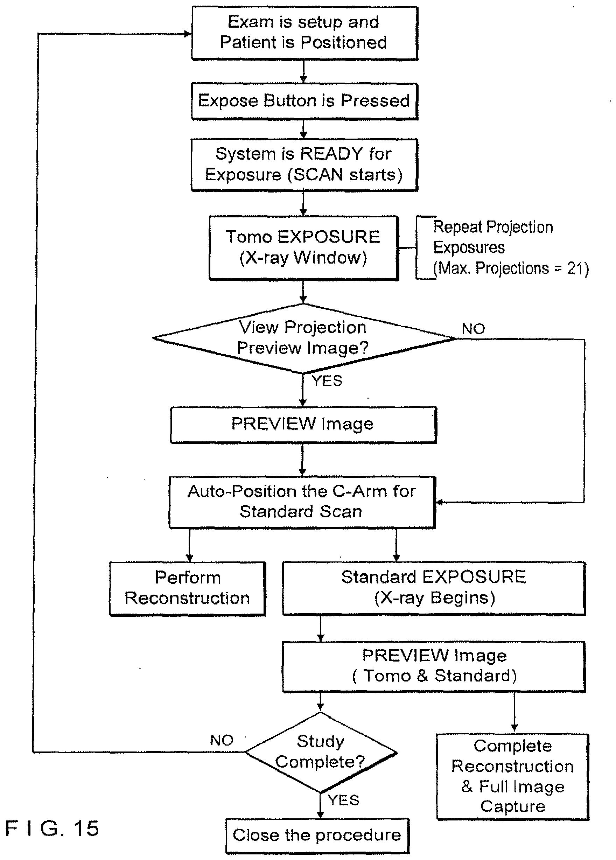

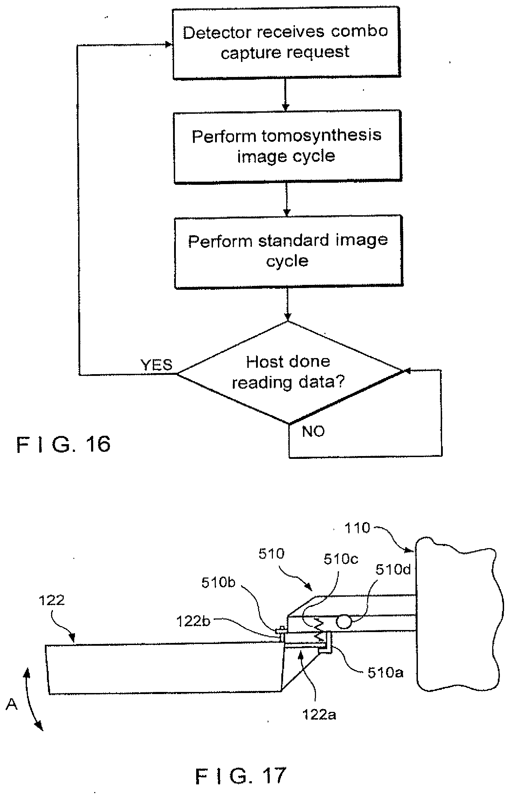

In a combination mode, during a single compression of the patient's breast the system takes a conventional mammogram and tomosynthesis images. In this mode, while the breast remains compressed in compression arm assembly 110, (1) tube arm assembly 106 sweeps and x-ray receptor 502 rocks, each through an appropriate angle, and exposures are taken for tomosynthesis images, and (2) a standard mammogram is taken. The standard mammogram can be taken at a 0° relative angle between tube arm assembly 106 and a normal to the imaging plane of x-ray receptor 502, and can be taken before or after the tomosynthesis images are taken or between the taking of two successive tomosynthesis images. Typically, each tomosynthesis image utilizes substantially lower x-ray dose than the standard mammogram. For example, the total x-ray dosage for tomosynthesis imaging in one sweep of tube arm assembly 106 can be approximately the same as that for a single standard mammogram, or up to approximately three times that dosage. The relationship between the two dosages can be user-selected. Figure 15 illustrates an example of workflow for the combination mode, and Fig. 16 illustrates an example of the operation of detector subsystem 117 in that mode. Again, these are examples, and different steps or orders of steps can be used instead. For example, a preferred approach may be to take the standard mammogram first, then move arm 106 to one end of its rotational range for tomosynthesis and take the tomosynthesis images. The order in which the two types of images are taken may be optimized such that the overall imaging time is minimized, and an order that achieves such minimization can be the preferred order.
The exposure (tube current mA, tube voltage kVp, and exposure length msec) techniques for the standard mammogram and the tomosynthesis exposures can be set manually, by using automatic methods or using the methods described above. If the standard mammogram is taken first, its exposure techniques can be used to set an optimal technique for the subsequent tomosynthesis images, and vice versa. The exposure technique can be modified dynamically, if the software senses that the signal reaching the image receptor is either too low or too high and adjust subsequent exposures as needed.

In a stereotactic mode, during a single compression of the patient's breast at least two images of taken, for example one at (+15)° angle and one at (-15°) angle of tube arm assembly 106 relative to compression arm assembly 110, although other angles can be used and more images can be taken. X-ray receptor 502 can remain in place for this procedure, or can be rocked through a selected angle, for example through an angle sufficient to maintain the same orientation of the imaging surface of receptor 502 relative to tube arm assembly 106. A spacer 1002 can be used for magnification. If x-ray receptor 502 remains in place despite rotation of arm 106, or if spacer 1002 is used, anti-scatter grid 504 is fully retracted; if x-ray receptor 502 maintains its orientation relative to tube arm assembly 106 and not spacer 1002 is used, anti-scatter grid 504 need not be retracted. As is known in the art, the two or more images can be used to identify the location of a lesion, so that needle biopsy can be used, for example with an upright needle biopsy station 412 (Fig. 4) in a manner similar to that used with the commercially available Selenia™ system and StereoLoc II™. A compression paddle 122 appropriate for needle biopsy typically is used when taking the stereotactic images. Alternatively, some or all of the images taken in the tomosynthesis mode and/or in the combined mode can be used to identify the location of a lesion for biopsy, in which case a compression paddle 122 appropriate for the purpose typically is used when taking the images.

In needle localization mode, x-ray images can be taken after a biopsy or other needle is inserted into the compressed breast. For this purpose, imaging such as in the stereotactic mode, the tomosynthesis mode, or the combined mode can be used.

In the disclosed system, compression paddle 122 is movable laterally, as generally described in co-pending U.S. Patent Application Publication No. 2005/0063509 A1. In addition, compression paddle 122 can pivot about an axis along the patient's chest wall to conform the breast shape in certain procedures, as discussed in said U.S. Patent 5,706,327. However, in the system of this patent specification compression paddle 122 is mounted differently and moves in a different manner.

As illustrated in Figs. 5 and 17, compression paddle 122 is removably mounted to a support 510 that moves up and down compression arm assembly 110 as needed for breast compression. To mount compression paddle 122 onto 510, a projection compression paddle 122a of the paddle engages a projection 510a of the support, and a projection 122b of the paddle latches onto projection 510b of the support. Projection 510a is springloaded, such as by a spring schematically illustrates at 510c to allow for pivoting compression paddle 122 about an axis where it latches onto 510, as illustrated by arrow A, for better conformance with the compressed breast in some imaging protocols. Other imaging protocols may require compression paddle 122 not to pivot, in which case projection 510a is locked in place by a locking mechanism in 510 (not shown) to keep compression paddle 122 in place relative to support 510. The locking mechanism can be manually set to a lock position, and manually unlocked by the operator. Alternatively, the locking mechanism can be controlled through an operator input at gantry 100 or work-station 102. A sensing mechanism can be included to sense whether compression paddle 122 is locked against pivoting, to provide information that work-station 102 can use for setting imaging protocols such as for automated breast compression and automated exposure methods. Two knobs 510d, one on each lateral side of support 510, can be manually rotated to move projection 510b and thus compression paddle 122 laterally such that it compress a breast that is not centered laterally on upper surface 116, for example for MLO imaging. Each knob 510d can operate a mechanism such as an endless screw rotating in a nut secured to projection 510b. Alternatively, or in addition, projection 510b and thus compression paddle 122 can be driven laterally by a motor, under control of operator switches or other interface at gantry 100 or at work-station 102, or automatically positioned laterally under computer control.

Importantly, compression paddle 122 is driven for lateral movement by components that are a part of support 510. Thus, compression paddle 122 can be simple structure, and can even be disposable, with a new one used for each patient or for only a few patients. This can simplify and reduce the cost of using the system, because an imaging facility usually stocks a number of different paddles for different purposes. If the lateral movement mechanism is integral with a compression paddle, the paddle assembly is considerably larger, heavier and more expensive. But with a compression paddle 122 that relies for lateral movement on support 510, and is easily mounted by hand and without tools to support 510, by sliding compression paddle 122a into projection 510a and latching projection paddle 122b onto projection 510b, and is easily removed by reversing the process, the expense of keeping a number of different compression paddles in stock or replacing paddles with new ones is greatly reduced, as are the time and convenience when changing from one type of compression paddle to another. Compression paddle 122 can include a bar code that is automatically read by a bar code reader in support 510, to keep work-station 102 informed of the paddle currently mounted to support 510, for use in automating imaging protocols. For example, the bar code information can be checked to ensure through computer processing that the type of paddle that is currently mounted on support 510 matches the imaging that will be commanded, and the information from the sensor for whether compression paddle 122 is locked in non-tilting mode can be used to automatically make adjustments for compression height to ensure accurate automatic x-ray exposure operation. Further, the bar code information identifying the paddle can be used to automatically set collimation in x-ray tube assembly 108 so that the x-ray beam matches the size and shape of the currently installed compression paddle 122.

Thus a system for multi-mode breast x-ray imaging that supports at least two imaging modes has been shown and described. Multiple different imaging modes may be used in a single breast compression or alternatively using temporally spaced compressions. The imaging modalities include, but are not limited to, mammography, dynamic imaging mode, diagnosis mode, tomosynthesis, combination mode and stereotactic mode. The system facilitates imaging protocols wherein at least one imaging mode differs from at least one other imaging mode for at least one imaging capture procedure selected from a group including, but are not limited to receptor motion, x-ray source location, anti-scatter grid use, exposure control and patient shielding.

The system further supports a combination imaging mode wherein images are captured using at least two imaging modes during a single image scan and wherein each of the at least two imaging modes differs by at least one image capture procedure during the single scan. Such an arrangement facilitates fast capture of a plurality of images of different types without decompression of a patient's breast. As a result, the quantity and quality of information available for screening and diagnosis is substantially increased without concomitant increase in examination time or patient discomfort.

The above specific examples and embodiments are illustrative, and many variations can be introduced on these examples and embodiments. For example, elements and/or features of different illustrative embodiments may be combined with each other and/or substituted for each other within the scope of the appended claims.

## Claims

1. A multi-mode breast x-ray imaging system comprising:
an x-ray source (108);
an x-ray detector, adapted to provide photon count;
a removable anti-scatter grid,
wherein the x-ray source and x-ray detector are configured to perform a single image scan which generates images during a single breast compression using at least two different imaging modes,
wherein in at least one of said imaging modes, the anti-scatter grid is removed,
and wherein the imaging system is arranged to apply exposure control by using a desired x-ray dose, to take (1912) a scout image and identify from the scout image (1914) the density of a patient's breast,
**characterised in that** the imaging system is further arranged to identify (1916) a detector target count using the identified density, wherein the detector target count relates a count of photons to said desired x-ray dose.

2. The multi-mode breast x-ray imaging system according to claim 1, wherein the x-ray detector is adapted to rock in at least one imaging mode and preferably is adapted to rock along an angular displacement that is less than an angular displacement of the x-ray source in the at least one imaging mode.

3. The multi-mode breast x-ray imaging system according to any one of the preceding claims, wherein a compression arm is disposed between the x-ray source and x-ray detector for compression of a patient's breast.

4. The multi-mode breast x-ray imaging system according to any one of the preceding claims, wherein the imaging modes differ with regard to an imaging procedure selected from a group including detector motion, exposure control and patient shielding.

5. The multi-mode breast x-ray imaging system of claim 4, wherein the patient shielding is removed in at least one imaging mode.

6. The multi-mode breast x-ray imaging system of claim 4, wherein a size of the patient shield differs for at least two imaging modes.

7. The multi-mode breast x-ray imaging system as claimed in claim 4 or claim 6, wherein the patient shield is positioned differently for at least two imaging modes.

## Patentansprüche

1. Multimodales Bildgebungssystem zur Röntgenuntersuchung der Brust, umfassend:
eine Röntgenstrahlenquelle (108);
einen Röntgenstrahlendetektor, der geeignet ist um eine Photonenanzahl bereitzustellen;
ein abnehmbares Streustrahlenraster,
wobei die Röntgenstrahlenquelle und der Röntgenstrahlendetektor konfiguriert sind um einen Einzelbild-Scan auszuführen der während der Kompression einer einzigen Brust unter Verwendung von mindestens zwei verschiedenen Bildgebungsmodi Bilder generiert,
wobei in mindestens einem der Bildgebungsmodi das Streustrahlenraster abgenommen ist,
und wobei das Bildgebungssystem eingerichtet ist um eine Belichtungssteuerung unter Verwendung einer gewünschten Röntgendosis anzuwenden, um ein Scout-Bild aufzunehmen (1912) und aus dem Scout-Bild (1914) die Dichte der Brust eines Patienten zu identifizieren,
**dadurch gekennzeichnet dass** das Bildgebungssystem ferner eingerichtet ist um eine Detektorzielanzahl unter Verwendung der identifizierten Dichte zu identifizieren (1916), wobei die Detektorzielanzahl einen Zusammenhang zwischen der Anzahl von Photonen mit der gewünschten Röntgendosis herstellt.

2. Multimodales Bildgebungssystem zur Röntgenuntersuchung der Brust gemäß Anspruch 1, wobei der Röntgenstrahlendetektor geeignet ist um in mindestens einem Bildgebungsmodus zu schwanken und bevorzugt geeignet ist um entlang einer Winkelverlagerung zu schwanken die geringer als eine Winkelverlagerung der Röntgenstrahlenquelle in dem mindestens einen Bildgebungsmodus ist.

3. Multimodales Bildgebungssystem zur Röntgenuntersuchung der Brust gemäß einem der vorhergehenden Ansprüche, wobei ein Kompressionsarm zwischen der Röntgenstrahlenquelle und dem Röntgenstrahlendetektor zur Kompression der Brust einer Patientin angeordnet ist.

4. Multimodales Bildgebungssystem zur Röntgenuntersuchung der Brust gemäß einem der vorhergehenden Ansprüche, wobei sich die Bildgebungsmodi in Bezug auf einen Bildgebungsarbeitsablauf unterscheiden, der aus einer Gruppe ausgewählt wird, die Detektorbewegung, Belichtungssteuerung und Patientenabschirmung umfasst.

5. Multimodales Bildgebungssystem zur Röntgenuntersuchung der Brust gemäß Anspruch 4, wobei die Patientenabschirmung in mindestens einem Bildgebungsmodus abgenommen ist.

6. Multimodales Bildgebungssystem zur Röntgenuntersuchung der Brust gemäß Anspruch 4, wobei eine Größe des Patientenschutzschildes für mindestens zwei Bildgebungsmodi unterschiedlich ist.

7. Multimodales Bildgebungssystem zur Röntgenuntersuchung der Brust gemäß Anspruch 4 oder 6, wobei das Patientenschutzschild für mindestens zwei Bildgebungsmodi unterschiedlich positioniert ist.

## Revendications

1. Système d'imagerie par rayons X pour mammographie multi-mode comprenant:
une source de rayons X (108);
un détecteur de rayons X, conçu pour fournir un comptage de photons;
une grille anti-diffusion amovible,
dans lequel la source de rayons X et le détecteur de rayons X sont configurés pour réaliser un unique balayage d'image qui génère des images durant une unique compression du sein à l'aide d'au moins deux modes d'imagerie différents,
dans lequel dans au moins l'un desdits modes d'imagerie, la grille anti-diffusion est retirée,
et dans lequel le système d'imagerie est agencé pour appliquer un contrôle d'exposition par utilisation d'une dose de rayons X souhaitée, pour prendre (1912) une image de repérage et identifier à partir de l'image de repérage (1914) la densité d'un sein de patiente,
**caractérisé par le fait que** le système d'imagerie est en outre agencé pour identifier (1916) un comptage cible de détecteur en utilisant la densité identifiée, le comptage cible de détecteur portant sur un comptage de photons à ladite dose de rayons X souhaitée.

2. Système d'imagerie par rayons X pour mammographie multi-mode selon la revendication 1, dans lequel le détecteur de rayons X est conçu pour se balancer dans au moins un mode d'imagerie et de préférence est conçu pour se balancer le long d'un déplacement angulaire qui est inférieur à un déplacement angulaire de la source de rayons X dans l'au moins un mode d'imagerie.

3. Système d'imagerie par rayons X pour mammographie multi-mode selon l'une quelconque des revendications précédentes, dans lequel un bras de compression est disposé entre la source de rayons X et le détecteur de rayons X pour une compression d'un sein de patiente.

4. Système d'imagerie par rayons X pour mammographie multi-mode selon l'une quelconque des revendications précédentes, dans lequel les modes d'imagerie diffèrent en ce qui concerne une procédure d'imagerie choisie parmi un groupe comprenant un mouvement de détecteur, un contrôle d'exposition et une protection de patient.

5. Système d'imagerie par rayons X pour mammographie multi-mode selon la revendication 4, dans lequel la protection de patiente est retirée dans au moins un mode d'imagerie.

6. Système d'imagerie par rayons X pour mammographie multi-mode selon la revendication 4, dans lequel une dimension de l'écran pour patient diffère pour au moins deux modes d'imagerie.

7. Système d'imagerie par rayons X pour mammographie multi-mode selon la revendication 4 ou la revendication 6, dans lequel l'écran pour patient est positionné différemment pour au moins deux modes d'imagerie.
